# EUROPEAN PATENT SPECIFICATION

(11) **EP 4 135 756 B1**
(45) Date of publication and mention of the grant of the patent: **27.03.2024**
(21) Application number: 21718158.5
(22) Date of filing: 19.04.2021
(51) Int. Cl.: A61K 38/57, C07K 14/81, A61P 11/00, A61P 31/12, A61P 31/14, A61P 31/16, C07K 14/44

(54) **C1 ESTERASE INHIBITOR TO TREAT CORONAVIRAL INFECTION-RELATED RESPIRATORY DISTRESS**
C1-ESTERASE-INHIBITOR ZUR BEHANDLUNG VON ATEMNOTSYNDROM IN ZUSAMMENHANG MIT CORONAVIRUSINFEKTIONEN
INHIBITEUR DE C1 ESTÉRASE POUR TRAITER UNE DÉTRESSE RESPIRATOIRE LIÉE À UNE INFECTION CORONAVIRALE

(30) Priority: 17.04.2020 EP 20170253; 17.04.2020 US 202063011738 P
(43) Date of publication of application: 22.02.2023
(73) Proprietor: Pharming Intellectual Property B.V., 2333 CR Leiden (NL)
(72) Inventor: GIANNETTI, Bruno, 2333 CR Leiden (NL); RELAN, Anurag, 2333 CR Leiden (NL); SCHAALE-MAASS, Juergen Ulrich, 2333 CR Leiden (NL)
(74) Representative: Nederlandsch Octrooibureau
(86) International application number: PCT/EP2021/060098
(87) International publication number: WO 2021/209644

(56) References cited:
- WO-A1-2005/016375
- US-A1- 2006 233 776
- US-A1- 2007 185 011
- US-A1- 2016 058 850
- US-B1- 7 053 176

## Description

### TECHNICAL FIELD

The disclosure relates to treatment of virus-related respiratory distress, particularly methods for treating such distress by administering C1 esterase inhibitor (C1INH).

### BACKGROUND

The complement system is an integral part of the innate immune system and consists of a number of distinct plasma proteins that act as a first line of defense inducing an inflammatory response after opsonization of pathogens and dying cells (Walport 2001, N.E.J.M. 344:1058-1066; Walport 2001, N.E.J.M. 344:1140-1144). The complement system and particularly the lectin pathway has been found to interact with and be involved in the clearance of a number of viruses (Thielens 2002, Immunobiology 205:563-574; Kase et al. 1999, Immunology 97:385-392; Bibert et al. 2019, PLoS Pathol. 15; Schiela et al., Front. Immunology 9:2177; Bermejo-Jambrina et al., Front. Immunology 9:590). However, unregulated complement activation may play a role in the pathogenesis of acute lung injury (ALI) induced by viruses including influenza and Severe Acute Respiratory Syndrome (SARS) corona viruses (CoV). One experimental model suggests that the complement system may be implicated in SARS-CoV-induced lung disease via regulation of the systemic proinflammatory response. Complement deficient mice infected with SARS-CoV were affected less severely and showed a reduced lung involvement and lower local and systemic cytokine levels compared to control mice (Gralinski et al. 2018, mBio 9(5)). In line with that finding, inhibition of complement C5a signaling alleviated lung damage in a MERS-CoV mouse model (Jiang et al. 2018, Emerg. Microbes Infect. 7:77) and an influenza H7N9 monkey model (Sun et al. 2015, Clin. Infect. Dis. 60:586-595). The same was also observed in a mouse model of severe avian influenza infection when inhibiting the complement cascade at an earlier step (C3a) (Sun et al., 2013, Am. J. Resp. Cell Mol. Bio. 49:221-230).

C1 esterase inhibitor (C1INH), a member of the serpin superfamily of serine-protease inhibitors, is an acute-phase protein that has manifold targets and biological functions, such as inhibition of leucocytes and interactions with endothelial cells and microorganisms. Further, C1INH is the natural and strong inhibitor of the classical and lectin pathway of complement (earliest step of complement activation) and factor XII and plasma kallikrein of the contact system. C1INH is encoded by a single gene (*SERPING1*) on chromosome 11 that consists of 8 exons and 7 introns. The entire genomic sequence is known and codes for a protein of 500 amino acids, including a 22 amino acid signal sequence (Carter P. et al. 1988, Euro. J. Biochem. 173; 163). Plasma C1INH is a glycoprotein of approximately 105 kDa and is heavily glycosylated, with up to 50% of its molecular mass consisting of carbohydrate.

Purified and recombinant forms of C1INH have been approved for use as, and are currently used as, a therapeutic. Currently, four C1INH therapeutic preparations are available, three of them plasma-derived (Cinryze^{®}, Berinert^{®}, and Haegarda^{®}) and one recombinant, i.e., recombinant human C1INH (Ruconest^{®}, Pharming, Leiden, The Netherlands). Recombinant human C1INH shares an identical protein structure with plasma-derived C1INH but has a different glycosylation pattern (containing abundant oligomannose residues), which is responsible for a shorter half-life than plasma-derived C1INH (3h vs. 30h) (Davis and Bernstein 2011, Clinical Risk Management 7:265-273; van Veen et al. 2012, J. Biotechnology 162:319-326). Despite the rather broad interference with several cascades and targets, major adverse events or unique toxicities have not been demonstrated in previous studies, with the exception of a potential risk of allergic reactions in patients with rabbit allergy receiving recombinant human C1INH.

C1INH therapeutic preparations are used to treat hereditary angioedema (HAE). HAE is most often caused by a genetic defect in *SERPING1* that leads to loss of C1INH expression or expression of a functionally-deficient C1INH. HAE is defined by recurrent episodes of angioedema without urticaria or pruritus, and treatment with C1INH is able to alleviate these acute symptoms by replacing the deficient or absent C1INH. Long-term prophylaxis with certain C1INH preparations is also used as a treatment for HAE, with the goal of preventing or minimizing the number and severity of angioedema attacks.

C1INH has been identified as being useful for treat other diseases or conditions in which classical pathway complement activity (C1 component) and/or contact system activity (factor XIIa, kallikrein, factor XIa) contributes to undesired immune or inflammatory responses (US 2005/0223416; Caliezi et al. 2000, Pharm. Rev. 52(1):91-112). For example, C1INH has been proposed for use as a therapeutic for reducing ischemia-reperfusion injury (US 8,071,532); as a therapeutic for preventing antibody-mediated rejection of transplanted organs (WO 2015/077543); and as a therapeutic for treating and preventing pre-eclampsia (WO 2019/166556).

There remains a need in the field for a method of treating virus-related respiratory disorders. The invention disclosed herein meets that need with methods comprising administering a therapeutically effective amount of C1INH to patients suffering from virus-related respiratory distress.

### SUMMARY

The disclosure of this application is directed to a therapeutically effective amount of a C1 esterase inhibitor (C1INH) for use in the treatment of a patient suffering from respiratory distress wherein the respiratory distress is related to a coronavirus infection. In some embodiments, the patient is suffering from pneumonia. In some embodiments, the patient is a human.

For example, in particular embodiments the coronavirus is SARS-CoV-2. In some embodiments, the patient has antibodies against SARS-CoV-2. In some embodiments, the respiratory distress is related to the 2019 coronavirus disease ("COVID-19").

In some embodiments, the patient is suffering from hypoxia. In some embodiments, the patient requires oxygen support. In some embodiments, the patient requires a ventilator. In some embodiments, the C1INH is administered before the patient requires a ventilator. But in other embodiments, the C1INH is administered to a patient already on a ventilator.

In some embodiments, the administered C1INH has an amino acid sequence identical or similar to the amino acid sequence of endogenous human C1INH. In some embodiments, the C1INH is recombinant human C1INH. In some embodiments, the C1INH has a plasma half-life of less than 6 hours. In some embodiments, the C1INH has a different level of sialic acid residues compared to endogenous plasma-derived human C1INH. In some embodiments, the C1INH is produced in a transgenic animal or in a recombinant cell culture system. In some embodiments, the C1INH is produced in a transgenic rabbit. And in particular embodiments, the CIINH is Ruconest^{®}. Alternatively, in some embodiments, the C1INH is plasma-derived human Cl esterase inhibitor.

According to various embodiments of the invention, the CIINH can be administered by a variety of biological routes. For example, in some embodiments, the C1INH is administered intravenously. In some embodiments, the C1INH is administered subcutaneously. And in some embodiments, the CIINH is administered intramuscularly. In some embodiments, the C1INH is self-administered.

The C1INH can be administered at a range of doses and according to a variety of dosing schedules. In some embodiments, the C1INH is administered at a dose of at least about 25 U/kg body weight of the patient. In some embodiments, the C1INH is administered at a dose of at least about 50 U/kg body weight of the patient. In some embodiments, the C1INH is administered at a dose of at least about 60 U/kg body weight of the patient. In some embodiments, the C1INH is administered at an initial dose of at least about 100 U/kg, followed by at least about 50 U/kg C1INH every eight hours over a period of at least 72 hours. In some embodiments, the C1INH is administered at an initial dose of at least about 100 U/kg, followed by at least about 50 U/kg C1INH every twelve hours over a period of at least 72 hours. In some embodiments, the C1INH is administered every six hours or every 12 hours until the clinical symptoms and the inflammatory markers have decreased below 50% of the initial pathological status or reached normal values.

In some embodiments, the C1INH is administered at a dose of about 4200 units C1INH about every twelve hours over a period of at least about 96 hours. In some embodiments, the C1INH is administered at a dose of about 50 U/kg of C1INH about every twelve hours over a period of at least about 96 hours. In some embodiments, the weight of the patient determines the dosing strategy, such that the C1INH is administered at a dose of about 4200 units C1INH about every twelve hours over a period of at least about 96 hours if the patient weighs more than 84 kg, or is administered at a dose of about 50 U/kg of C1INH about every twelve hours over a period of at least about 96 hours if the patient weighs up to 84 kg. In some embodiments, the C1INH is administered at an initial dose of at least about 8400 units C1INH, followed by at least about 4200 units C1INH every eight hours over a period of at least 72 hours. In some embodiments, the C1INH is administered at an initial dose of at least about 8400 units C1INH, followed by at least about 4200 units C1INH every twelve hours over a period of at least 36 hours. In some embodiments, the C1INH is administered every eight hours or every twelve hours until the clinical symptoms and the inflammatory markers have decreased below 50% of the initial pathological status or reached normal values. In some embodiments, the clinical symptoms used to determine how long to continue treatment are selected from the group consisting of oxygen requirements, radiographic signs, respiratory rate, and a combination thereof. In some embodiments, the inflammatory markers used to determine how long to continue treatment are selected from the group consisting of CRP, D-dimers, IL-6, ferritin, and a combination thereof. In some embodiments, the treatment results in defervescence within 24 hours or within 48 hours.

In some embodiments, the patient is administered a pharmaceutical composition comprising C1INH and a pharmaceutically acceptable carrier. In some embodiments, the patient is administered one or more therapeutics in addition to C1INH. In some embodiments, the one or more additional therapeutics are selected from the group consisting of hydroxychloroquine, chloroquine, remdesivir, umifenovir, baloxavir, favipiravir, lopinavir, ritonavir, a corticosteroid, tocilizumab, siltuximab, sarilumab, eculizumab, gimsilumab, antibodies directed against components of the complement pathway, antibodies directed against components of the contact pathway, antibodies directed against the components of the lectin pathway, and combinations thereof. In some embodiments, the additional administered therapeutic is an antiviral agent. In some embodiments, the additional administered therapeutic is hydroxychloroquine or chloroquine. In some embodiments, the additional administered therapeutic is remdesivir.

In some embodiments, the patient is administered a therapeutic antibody in addition to C1INH. In some embodiments, the administered therapeutic antibody binds to an antigen present on a coronavirus. In some embodiments, the administered therapeutic antibody binds to an antigen present on SARS-CoV-2. In some embodiments, the administered therapeutic antibody is an antibody directed against components or structures of the complement system. In some embodiments, the administered therapeutic antibody binds to kallikrein. In some embodiments, the administered therapeutic antibody binds to bradykinin. In some embodiments, the administered therapeutic antibody binds to IL-6 receptors. In some embodiments, the administered therapeutic antibody is tocilizumab. In some embodiments, the administered therapeutic antibody binds to C5. In some embodiments, the administered therapeutic antibody is eculizumab. In some embodiments, the administered therapeutic antibody binds to C5a. In some embodiments, the administered therapeutic antibody is gimsilumab.

### BRIEF DESCRIPTION OF THE FIGURES

**Figure** 1 is a table containing the clinical characteristics of five SARS-CoV-2 infected patients who were treated with C1INH. The presented clinical characteristics are those measured at the time that C1INH treatment began in the patients in question.
**Figure** 2 is a table containing reference values for the normal range of parameters assessed and listed in Figure 1.
**Figure** 3 shows chest CT scans of the five patients before administration of C1INH showing ground-glass opacities involving multiple segments of both lungs with subpleural predominance and occasionally consolidations (e.g. in patient 4). The CT scans of the chest demonstrated moderate to severe pneumonia prior to administration of C1INH.
**Figures 4A, 4B** and **4C** are diagrams depicting dosing schedules for administering C1INH to a patient suffering from virus-related respiratory distress. To start treatment in the schedules depicted in Figure 4A and 4B, 8400 Units of C1INH are administered to the patient. Subsequently, 4200 Units of C1INH are administered to the patient either every twelve hours (Figure 4A) or every eight hours (Figure 4B) for the illustrated period. In the schedule depicted in Figure 4C, either 4200 U (weights over 84 kg) or 50 U/kg (weights up to 84 kg) are administered to the patient every 12 hours.
**Figure 5** shows the temporal change of body temperature following C1INH administration to five COVID-19 patients. Day 0 denotes the day of first C1INH administration. Following C1INH administration, decreased body temperature was observed in four of five patients within two days.
**Figures 6A to 6D** show the temporal changes of inflammatory markers following C1INH administration to five COVID-19 patients. Day 0 denotes the day of first C1INH administration. The inflammatory markers depicted are C-reactive protein (CRP) (Figure 6A), LDH (Figure 6B), ferritin (Figure 6C), and IL-6 (Figure 6D).
**Figures 7A to 7D** show the temporal changes of complement factors following C1INH administration to five COVID-19 patients. Day 0 denotes the day of first C1INH administration. The complement factors depicted are C1INH antigenic concentration (Figure 7A), CH50 (Figure 7B), complement component C3 (Figure 7C), and complement component C4 (Figure 7D).
**Figures 8A to 8D** show the temporal change of D-Dimer (Figure 8A), fibrinogen (Figure 8B), platelets (Figure 8C) and lymphocytes (Figure 8D) following the administration of C1INH to five COVID-19 patients. Day 0 denotes the day of first C1INH administration.
**Figure 9** shows the temporal change of alanine aminotransferase (ALT) following C1INH administration to five COVID-19 patients. Day 0 denotes the day of first C1INH administration.

### DETAILED DESCRIPTION

The present disclosure relates to methods of treating viral infection-related respiratory distress comprising administering C1INH.

### I. Definitions of general terms and expressions

In order that the present disclosure can be more readily understood, certain terms are first defined. As used in this application, except as otherwise expressly provided herein, each of the following terms shall have the meaning set forth below. Additional definitions are set forth throughout the application. In case of conflict, the present application including the definitions will control. Unless otherwise required by context, singular terms shall include pluralities and plural terms shall include the singular.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Although methods and materials similar or equivalent to those described herein can be used in practice or testing of the present disclosure, suitable methods and materials are described below. The materials, methods and examples are illustrative only and are not intended to be limiting. Other features and advantages of the disclosure will be apparent from the detailed description and from the claims.

The terms "administer", "administering", "administration", and the like, as used herein, refer to methods that may be used to enable delivery of a therapeutic, e.g., C1INH, to the desired site of biological action. Administration techniques that can be employed with the agents and methods described herein are found in e.g., Goodman and Gilman, The Pharmacological Basis of Therapeutics, current edition, Pergamon; and Remington's, Pharmaceutical Sciences, current edition, Mack Publishing Co., Easton, Pa. Administration refers to the physical introduction of a composition comprising a therapeutic agent to a subject, using any of the various methods and delivery systems known to those skilled in the art. Exemplary routes of administration for the formulations disclosed herein include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as *in vivo* electroporation. In some embodiments, the formulation is administered via a non-parenteral route, such as orally. Other non-parenteral routes include a topical, epidermal or mucosal route of administration, for example, intranasally, vaginally, rectally, sublingually or topically. Administering can also be performed, for example, once, a plurality of times, and/or over one or more extended periods.

The term "antibody" means an immunoglobulin molecule that recognizes and specifically binds to a target, such as a protein, polypeptide, peptide, carbohydrate, polynucleotide, lipid, or combinations of the foregoing. As used herein, the term "antibody" encompasses polyclonal antibodies, monoclonal antibodies, chimeric antibodies, humanized antibodies, fully human antibodies, recombinant antibodies, bispecific antibodies, fusion proteins comprising a full length antibody or fragments thereof, fragments of such antibodies, and any other modified immunoglobulin molecule so long as it exhibits the desired biological activity. An antibody can be of any the five major classes of immunoglobulins: IgA, IgD, IgE, IgG, and IgM, or subclasses (isotypes) thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well known subunit structures and three-dimensional configurations. Antibodies can be naked or conjugated to other molecules such as toxins, radioisotopes, etc.

Administration "in combination with" one or more further therapeutic agents includes simultaneous (concurrent) or consecutive administration in any order.

The combination therapy can provide "synergy," i.e., the effect achieved when the active agents used together is greater than the sum of the effects that result from using the active agents separately. A synergistic effect can be attained when the active agents are: (1) co-formulated and administered or delivered simultaneously in a combined, unit dosage formulation; (2) delivered serially, by alternation, or in parallel as separate formulations; or (3) by some other regimen. When delivered in alternation therapy, a synergistic effect can be attained when the active agents are administered or delivered sequentially, e.g., by different injections in separate syringes. A "synergistic combination" produces an effect that is greater than the sum of the effects of the individual active agents of the combination. The combination therapy can provide an "additive" effect, i.e., the effect achieved when the active agents used together is equal to the sum of the effects the result from using the active agents separately. A synergistic effect can also be an effect that cannot be achieved by administration of any of the active agents as single agents.

The terms "C1 Inhibitor," "C1 esterase Inhibitor," "C1-INH" and "C1INH" refer to the proteins or fragments thereof that function as serine protease inhibitors to inhibit proteases associated with the complement system, such as proteases C1r and C1s as well as MASP-1 and MASP-2; with the kallikrein-kinin system, such as plasma kallikrein and factor Xlla; and with the coagulation system, such as factor XIa. In addition, C1INH can serve as an anti-inflammatory molecule that reduces the selectins-mediated leukocyte adhesion to endothelial cells. C1INH, as used herein, can be a native serine protease inhibitor or active fragment thereof, or it can comprise a recombinant peptide, a synthetic peptide, peptide mimetic, or peptide fragment that provides similar functional properties- e.g., the inhibition of proteases C1r and C1s, MASP-1, MASP-2, factor Xlla, and/or factor XIa. C1INH, as used herein, includes both plasma-derived C1INH (e.g., purified from human plasma) and recombinantly produced C1INH (e.g., produced in rabbits or cell culture system).

As used herein, the term "respiratory distress" refers to a condition in which a patient has to work harder to breathe and/or is not getting sufficient oxygen. Examples of objective signs of respiratory distress include, for example, increased respiratory rate, accessory muscle use, hypoxemia, hypercapnea, and lethargy. The term "virus-related respiratory distress" refers to respiratory distress in a patient that occurs in connection with or as a result of a viral infection in that patient.

As used herein, the term acute respiratory distress syndrome ("ARDS") refers to a type of respiratory distress characterized by widespread inflammation in the lungs. ARDS is clinically characterized by (1) lung injury of acute onset, within 1 week of an apparent clinical insult and with progression of respiratory symptoms, (2) bilateral opacities on chest imaging (chest radiograph or CT) not explained by other lung pathology (e.g. effusion, lobar/lung collapse, or nodules) (3) respiratory failure not explained by heart failure or volume overload, and (4) decreased PaO₂/FiO₂ ratio. A patient with an "ARDS-like syndrome" exhibits some but not all of the clinical characteristics of a patient with ARDS.

As used herein, the term "viral infection" refers to an infection caused by the presence of a virus in a patient. Symptoms related to a viral infection include both the direct effects of the virus and the effects on or changes within the body that occur as a result of the patient's body (e.g., its immune system) response to the virus.

As used herein, the term "coronavirus" refers to any of the existing or future members of viruses of the family *Coronaviridae.* One exemplary member of the coronavirus family is the severe acute respiratory syndrome coronavirus (SARS-CoV). Another member of the coronavirus family is the severe acute respiratory syndrome coronavirus 2 (SARS-CoV-2). SARS-CoV-2 is the virus responsible for the 2019 coronavirus disease (COVID-19). Like SARS-CoV and SARS-CoV-2, future members of the coronavirus family may use the Angiotensin-Converting Enzyme 2 (ACE-2) as receptor for cell entry.

As used herein, the terms "subject" and "patient" are used interchangeably. The subject can be an animal. In some embodiments, the subject is a mammal such as a non-human animal (e.g., cow, pig, horse, cat, dog, rat, mouse, monkey or other primate, etc.). In some embodiments, the subject is a cynomolgus monkey. In some embodiments, the subject is a human.

As used herein, the terms "treat," "treatment," "treating," or "amelioration" when used in reference to a disease, disorder or medical condition, refer to both therapeutic treatment and prophylactic or preventative measures, wherein the object is to prevent, reverse, alleviate, ameliorate, inhibit, lessen, slow down and/or stop the progression or severity of a symptom or condition. The term "treating" includes reducing or alleviating at least one adverse effect or symptom of a condition. Treatment is generally "effective" if one or more symptoms or clinical markers are reduced. Alternatively, treatment is "effective" if the progression of a disease, disorder or medical condition is reduced or halted. That is, "treatment" includes not just the improvement of symptoms or markers, but also a cessation or at least slowing of progress or worsening of symptoms that would be expected in the absence of treatment. Also, "treatment" can mean to pursue or obtain beneficial results, or lower the chances of the individual developing the condition even if the treatment is ultimately unsuccessful. Those in need of treatment include those already with the condition as well as those prone to have the condition or those in whom the condition is to be prevented.

The term "therapeutically effective amount" refers to an amount of a drug, e.g., C1INH, effective to achieve the desired therapeutic or prophylactic result. A therapeutically effective amount also refers to an amount effective, at dosages and for periods of time necessary, to achieve the desired therapeutic effect. As such, a therapeutically effective amount can be delivered in one or more administrations. A therapeutically effective amount can vary according to factors such as the disease state, age, and weight of the individual. In some instances, the desired result is treating a disease or disorder in a subject. In particular embodiments, the desired result is treating virus-related respiratory distress or a virus-related respiratory disorder.

Units, prefixes, and symbols are denoted in their Système International de Unites (SI) accepted form. Numeric ranges are inclusive of the numbers defining the range. The headings provided herein are not limitations of the various aspects of the disclosure, which can be had by reference to the specification as a whole. Accordingly, the terms defined immediately below are more fully defined by reference to the specification in its entirety.

As used in the present disclosure and claims, the singular forms "a," "an," and "the" include plural forms unless the context clearly dictates otherwise.

It is understood that wherever embodiments are described herein with the language "comprising," otherwise analogous embodiments described in terms of "consisting of" and/or "consisting essentially of" are also provided. In this disclosure, "comprises," "comprising," "containing" and "having" and the like can have the meaning ascribed to them in U.S. Patent law and can mean "includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

Unless specifically stated or obvious from context, as used herein, the term "or" is understood to be inclusive. The term "and/or" as used in a phrase such as "A and/or B" herein is intended to include both "A and B," "A or B," "A," and "B." Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

The term "about" refers to a value or composition that is within an acceptable error range for the particular value or composition as determined by one of ordinary skill in the art, which will depend in part on how the value or composition is measured or determined, i.e., the limitations of the measurement system. For example, "about" or "comprising essentially of" can mean within 1 or more than 1 standard deviation per the practice in the art. Alternatively, "about" or "comprising essentially of" can mean a range of up to 20%. Furthermore, particularly with respect to biological systems or processes, the terms can mean up to an order of magnitude or up to 5-fold of a value. When particular values or compositions are provided in the application and claims, unless otherwise stated, the meaning of "about" or "comprising essentially of" should be assumed to be within an acceptable error range for that particular value or composition.

Any compositions or methods provided herein can be combined with one or more of any of the other compositions and methods provided herein.

### II. C1 Esterase Inhibitors

In the embodiments of the invention, the C1 esterase inhibitor (C1INH) may be any C1INH known to the person skilled in the art. In some embodiments of the invention, the C1INH is a plasma-derived C1INH. In some embodiments of the invention, the C1INH is a recombinant C1INH. In some embodiments of the invention, the C1INH has an amino acid sequence that is identical to the amino acid sequence of human C1INH. In some embodiments, the C1INH has an amino acid sequence that is similar to the amino acid sequence of human C1INH (i.e., having an amino acid sequence at least 90% identical to human C1INH while retaining C1INH's functional activity. The recombinant C1INH can be any recombinant C1INH known the person skilled in the art. It may be produced recombinantly in microbial cells, such as tissue culture cells. The tissue culture cell can be a mammalian tissue culture cell, such as a Chinese Hamster Ovarian (CHO) cell or a human tissue culture cell (see e.g. WO 2016/081889, which is herein incorporated by reference). The recombinant C1INH can be produced in transgenic animals, such as in a transgenic non-human mammal. The recombinant C1INH can be produced in a mouse, goat, bovine, sheep, porcine or an animal from the order *Lagomorpha,* such as a *Leporadae,* including a rabbit. In an embodiment, the recombinant C1INH is one produced according to the methods in WO 2001/57079, which is referenced herein. In some embodiments of the invention, the C1INH is Ruconest^{®}.

In some embodiments of the invention, the C1INH is a modified C1INH as compared to human plasma-derived C1INH. It can be modified to modulate the plasma half-life of the C1INH. A specific modified C1INH is conjugated to enhance the plasma half-life. An exemplary conjugated C1INH to enhance half-life is a conjugated C1INH according to WO 2017/176798, which is herein incorporated by reference. In some embodiments, the conjugated C1INH is a polysialic acid (PSA)-conjugated C1INH, or a polyethylene glycol (PEG)-conjugated C1INH. The modification of the C1INH can be a modified carbohydrate structure as compared to human plasma-derived C1INH. A specific modified C1INH has a reduced level of terminal sialic acid residues as compared to plasma derived C1INH, wherein said reduced level of terminal sialic acid residues may result in a reduction of plasma half-life to less than 6 hours. A specific C1INH having a reduced level of terminal sialic acid residues as compared to plasma derived C1INH is a C1INH according to WO 2001/57079, WO 2004/100982 and WO 2007/073186 which are referenced herein.

### III. Methods of Administering Pharmaceutical Compositions Comprising C1INH

The C 1INH according to the invention can be administered as part of a pharmaceutical composition. In some embodiments, the pharmaceutical composition comprises C1INH and a pharmaceutically acceptable excipient. In some embodiments, the pharmaceutical composition comprises C1INH and a pharmaceutically acceptable carrier. In some embodiments, the pharmaceutical composition comprises C1INH and a pharmaceutically acceptable stabilizer. As such, provided herein are compositions comprising C1INH having the desired degree of purity in a physiologically acceptable carrier, excipient and/or stabilizer. Acceptable carriers, excipients, or stabilizers are nontoxic to recipients at the dosages and concentrations employed. Guidance on pharmaceutically acceptable carriers, excipients, and stabilizers can be found in, for example, Remington's Pharmaceutical Sciences, 22nd ed., Pharmaceutical Press (2012); Gennaro, Remington: The Science and Practice of Pharmacy with Facts and Comparisons: Drugfacts Plus, 20th ed. (2003); Ansel et al., Pharmaceutical Dosage Forms and Drug Delivery Systems, 7th ed., Lippencott Williams and Wilkins (2004); Kibbe et al., Handbook of Pharmaceutical Excipients, 3rd ed., Pharmaceutical Press (2000).

Pharmaceutical compositions according to the invention can be administered by any means known to the person skilled in the art, such as but not limited, to intravenous, transdermal and subcutaneous administration. Intravenous administration is extensively described in WO 2001/57079, WO 2004/100982 and WO 2007/073186. Subcutaneous administration can be performed as in WO 2014/145519, US 9616111 B2 and EP 2968434 B1, which are referenced herein.

As used herein, one unit (U) of C1 esterase inhibitor is the amount of C1INH present in 1 milliliter of human plasma. One such unit corresponds to approximately 275 microgram plasma-derived C1INH. In some embodiments, a therapeutically effective amount of C1INH is administered. The C1INH can be administered in a dose ranging from 25 units/kg body weight to 100 units/kg body weight per administration. In some embodiments, the C1INH can be administered in a dose ranging from about 50 units/kg body weight to about 100 units/kg body weight per administration. Per administration, the dose can be 25 units/kg body weight, 50 units/kg body weight, or 100 units/kg body weight. The total dose per administration can be, for example, 500 units, 600 units 700 units, 800 units, 900 units, 1000 units, 1100 units, 1200 units, 1300 units, 1400 units, 1500 units, 1600 units, 1700 units, 1800 units, 1900 units, 2000 units, 2100 units, 2200 units, 2300 units, 2400 units, 2500 units, 2600 units, 2700 units, 2800 units, 2900 units, 3000 units, 3500 units, 4000 units, 4200 units, 4500 units, 4900 units, 5000 units, 5600 units, 6000 units, 6300 units, 7000 units, 7500 units, 8000 units, 8400 units or 9000 units of C1 inhibitor.

In some embodiments, the compositions comprising C1INH are sterile. Sterile compositions can readily be created, for example, by filtration through sterile filtration membranes.

### IV. Methods of Treating Respiratory Distress

In some embodiments, the present invention provides methods of treating a patient suffering from respiratory distress related to a viral infection by administering a therapeutically effective amount of C1INH. The C1INH can be any of the variants of C1INH disclosed herein.

In some embodiments, the respiratory distress is ARDS. In some embodiments, the respiratory distress is atypical ARDS or an ARDS-like syndrome. In some embodiments, the respiratory distress is hypoxemia or hypoxia (defined as a PaO2/FiO2 of <300 mmHg). In some embodiments, the respiratory distress is pneumonia. In some embodiments, the respiratory distress is evidenced by increased pulmonary vascular permeability, increased inflammatory myeloid cells infiltrating the lung, or other lung pathologies indicative of reduced or poor lung function.

The viral infection is a coronavirus infection. For example, in some embodiments the coronavirus infection is a SARS-CoV infection, a SARS-CoV-2 infection, or MERS-CoV infection. In some embodiments, the coronavirus infection is a coronavirus that uses the Angiotensin-Converting Enzyme 2 (ACE-2) as a receptor for cell entry. In other embodiments, the viral infection is an Influenza infection. In other embodiments, the viral infection is an infection by a virus of unknown origin that induces respiratory distress.

Embodiments of the invention can begin administration of C1INH at any stage of a SARS-CoV-2 infection, as defined by Siddiqi et al. 2020, Journal of Heart and Lung Transplantation (https://www.jhltonline.org/article/S1053-2498(20)31473-X/fulltext). Accordingly, in some embodiments of the invention treatment begins in patients with a stage I (mild) SARS-CoV-2 infection. In some embodiments of the invention, treatment begins in patients with a stage II (moderate) SARS-CoV-2 infection. And in some embodiments of the invention, treatment begins in patients with a stage III (severe) SARS-CoV-2 infection. In some embodiments, treatment begins when a patient does not require oxygen support. In some embodiments, treatment begins when a patient does not require aid of a ventilator. In some embodiments, treatment begins at the time when, or after, a patient requires oxygen support (e.g., administration of oxygen to the patient). In some embodiments, treatment begins at the time when, or after, a patient is put on a ventilator.

In some embodiments, C1INH according to the invention is administered to the subject at least once a month, or at least once a week. In some embodiments, the C1INH is administered at least once, twice, three or four times a month. In some embodiments, the C1INH is administered at least once, twice, three, four, five, six or seven times a week. In some embodiments, the C1INH is administered every other day, daily, or twice a day. In some embodiments, after the initial administration the C1INH is administered once every two hours, once every three hours, once every four hours, once every five hours, once every six hours, once every seven hours, once every eight hours, once every nine hours, once every ten hours, once every eleven hours, or once every twelve hours.

In some embodiments, C1INH is administered only once. In some embodiments, C1INH treatment is continued for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, or longer as needed. In some embodiments, C1INH treatment is continued until the patients respiratory distress ceases. In some embodiments, C1INH treatment is continued until the viral infection receded, as shown by, for example, a decreased viral load. In some embodiments, CIINH treatment is continued until the viral infection is undetectable in the blood by real-time PCR. In some embodiments, CIINH treatment is continued until inflammatory markers such as CRP, D-dimers, IL-2, IL-6, IL-7, Ferritin, or GM-CSF return to baseline levels. In some embodiments, C1INH treatment is continued until improvement is observed by clinical signs such as reduced oxygen requirements, improved radiographic signs, or improved vital signs (e.g., respiratory rate).

In some embodiments, the method of treating a patient suffering from respiratory distress comprises administering C1INH in combination with one or more additional therapeutics. The additional therapeutic or therapeutics may be an anti-viral therapeutic. The additional therapeutic or therapeutics may target components of the complement, contact, or lectin pathway. Exemplary therapeutics that may be used in combination with C1INH include an angiotensin-converting enzyme (ACE) inhibitor, an angiotensin receptor blocker (ARB), hydroxychloroquine, chloroquine, remdesivir, umifenovir, baloxavir, favipiravir, lopinavir, ritonavir, a corticosteroid, tocilizumab, siltuximab, sarilumab, eculizumab, gimsilumab, antibodies directed against components of the complement pathway, antibodies directed against components of the contact pathway, antibodies directed against the components of the lectin pathway, and combinations thereof.

In some embodiments, the method treating a patient suffering from respiratory distress comprises administering both CIINH and a therapeutic antibody. In some embodiments, the therapeutic antibody binds to a component of the complement, contact, or lectin pathway. In some embodiments, the therapeutic antibody binds to kallikrein. In some embodiments, the therapeutic antibody binds to bradykinin. In some embodiments, the therapeutic antibody binds to IL-6 receptors (e.g., tocilizumab) or to IL-6 (e.g., siltuximab or sarilumab). In some embodiments, the therapeutic antibody binds to C5 (e.g., eculizumab). In some embodiments, the therapeutic antibody binds to C5a. In some embodiments, the therapeutic antibody binds to an antigen present on a coronavirus. In some embodiments, the therapeutic antibody binds to an antigen present on SARS-CoV-2. In some embodiments, the therapeutic antibody is gimsilumab.

The medical treatment described in the embodiments herein are formulated as: "A method of treatment of a patient suffering from respiratory distress, comprising administering a therapeutically effective amount of C1 esterase inhibitor (C1INH), wherein the respiratory distress is related to a viral infection." Equivalent formulations are listed in the section here below, such as: "a C1 esterase inhibitor (C1INH) for use in the treatment of a patient suffering from respiratory distress, wherein the respiratory distress is related to a viral infection and the treatment comprises administering a therapeutically effective amount of C1INH to the patient"; and "use of a C1 esterase inhibitor (C1INH) for the manufacture of a medicament for the treatment of a patient suffering from respiratory distress, wherein the respiratory distress is related to a viral infection and the treatment comprises administering a therapeutically effective amount of C1INH to the patient."

These equivalent formulations of the embodiments and their features can be used interchangeably.

176.

### EXAMPLES

### Example 1: Administration of C1INH to COVID-19 Patients Suffering From Respiratory Distress

Five patients with laboratory confirmed COVID-19, diagnosed using quantitative reverse transcriptase-polymerase chain reaction (PCR) were recruited for compassionate use of recombinant human C1INH (Ruconest^{®} Pharming, the Netherlands). All patients were diagnosed as having severe COVID-19, were at least 18 years old and showed evidence of persistent or progressive disease after 24 hours of standard treatment including hydroxychloroquin (HCQ) and lopinavir/ritonavir (lopinavir/r). Additional criteria included a C-reactive protein of at least 30 mg/L, and an oxygen saturation at rest in ambient air of ≤ 93%. The five patients ranged in age from 53 to 85 years old, and suffered from moderate to severe COVID-19 infection. The patients included four male patients and one female patient. The most common symptoms at presentation to the hospital were fatigue (4/5), fever (2/5), cough (4/5) and diarrhea (3/5). A National Early Warning Score 2 (NEWS) score of at least five was recorded in all patients on the day C1INH treatment which is consistent with an increased risk of acute deterioration and in-hospital mortality (Lee et al. 2018, Journal of Critical Care 47:222-226).

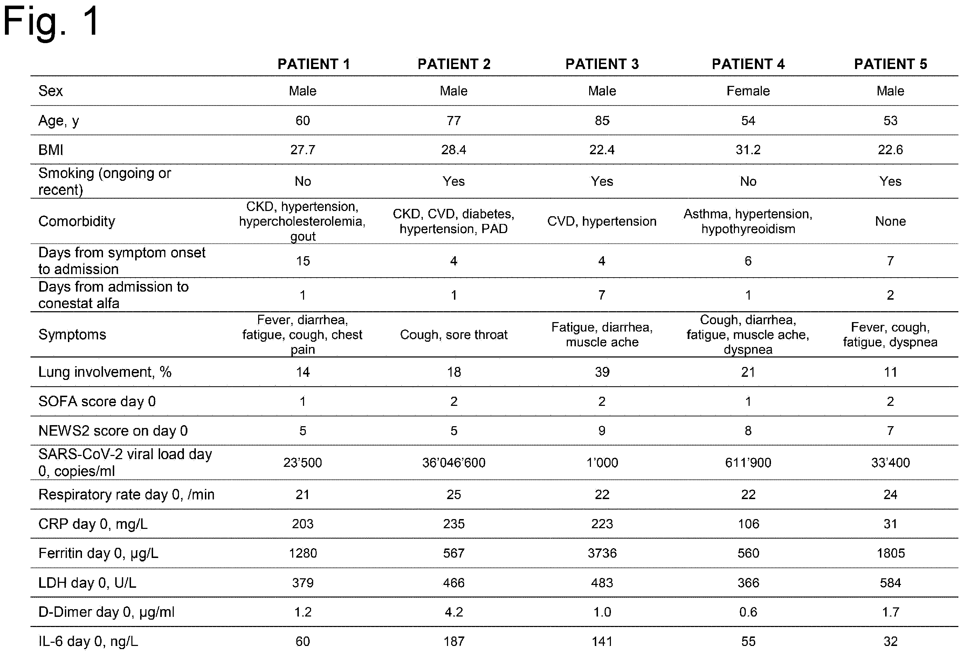

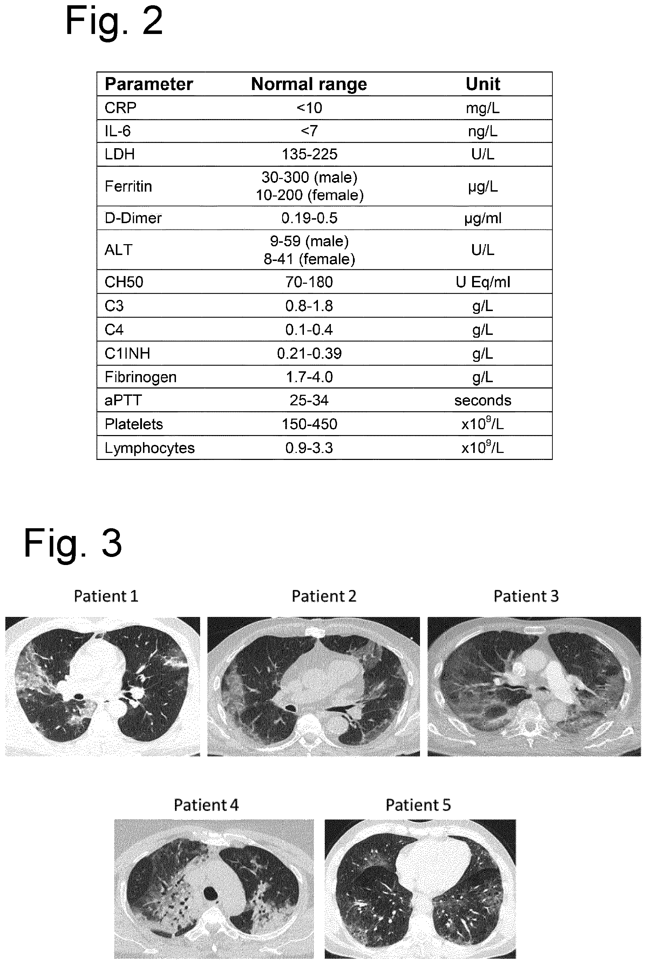

Clinical information for the five patients before and after administration of C1INH was obtained from reviewing the hospital information system and included the following: demographic data, days of admission from symptom onset, and presenting symptoms; data about various treatments, including mechanical ventilation, and steroids; clinical data, including body temperature, and Sequential Organ Failure Assessment (SOFA) score, laboratory data, including white blood cell count, lymphocyte count, chemistry panels assessing liver and kidney function, inflammatory factors including CRP, procalcitonin, and Interleukin (IL) -6, complement factors including CH50, C3 and C4, C1INH antigenic concentration; data from computer tomography scans of the chest; and information on tocilizumab or remdesivir treatment, mechanical ventilation, acute respiratory distress syndrome, bacterial pneumonia and multiple organ dysfunction syndrome. **Figure 1** contains a table with the clinical characteristics of the SARS-CoV-2 infected patients prior to the start of C1INH treatment. **Figure 2** provides reference values for the normal range of the clinical characteristics assessed. Blood tests were obtained every 1 to 2 days in the morning as per standard operating procedure of the hospital with additional complement measurements performed from the same blood collection.

The CT scans of the chest demonstrated moderate to severe pneumonia prior to administration of C1INH in all patients including bilateral ground-glass opacities and/or pulmonary consolidations with predominantly subpleural distribution and involving 11 to 39% of the lung parenchyma. **Figure 3** shows the pre-treatment CT scans.

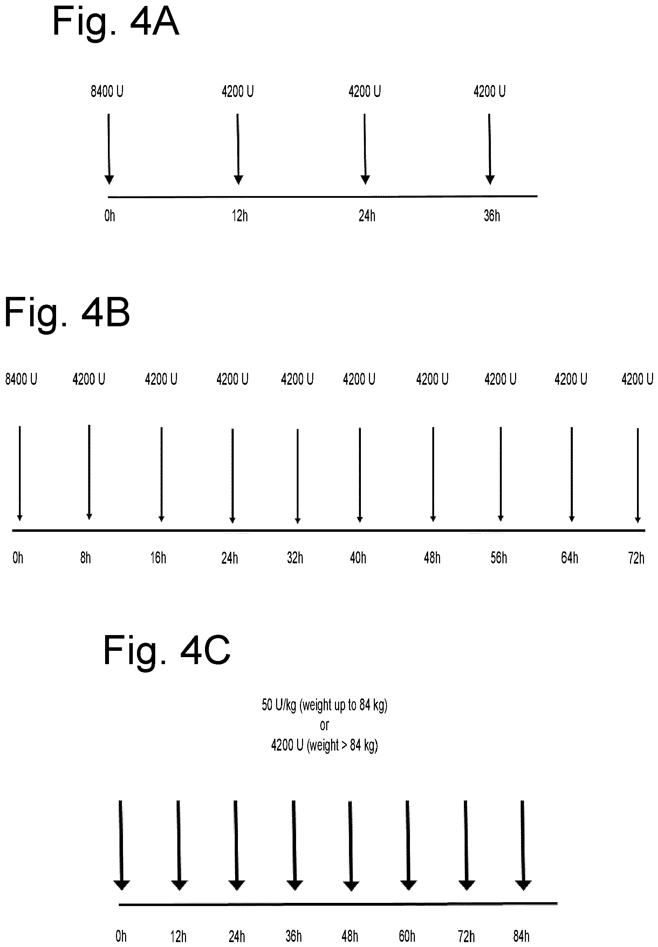

Each patient was administered an initial loading dose of 8400 units of C1INH via intravenous injection. Subsequently, each patient was administered 4200 units of C1INH every twelve hours over the next three days via intravenous injection. **Figure 4A** shows the dosing regimen used for all five patients. **Figure 4B** shows an alternative (unused) dosing schedule. All patients also received standard of care treatment for COVID-19 (HCQ plus lopinavir/r) during the period in which they were administered C1INH. Lopinavir/r was started in all patients, but ceased due to severe diarrhea (accompanied by acute on chronic renal failure in patient 2) in three patients before day five.

The primary objective of the study was to assess patients' clinical progress based on the World Health Organization's seven-point scale for outcome through at least day 7 (or time to improvement of one or two category). The steps in the scale were:
- Not hospitalized, no limitations on activities;
- Not hospitalized, limitation on activities;
- Hospitalized, not requiring supplemental oxygen;
- Hospitalized, requiring supplemental oxygen;
- Hospitalized, on non-invasive ventilation or high flow oxygen devices;
- Hospitalized, on invasive mechanical ventilation or extracorporeal membrane oxygenation (ECMO);
- Death.

The secondary objectives of the study were to assess patients' clinical progress based on:
- Disease progression;
- Changes in respiratory function;
- Need for intubation (proportion of patients requiring intubation after 14 days);
- Progression to ARDS.

Finally, the exploratory objectives of the study were to monitor the following variables:
- Safety: Participants will be monitored for adverse events and these will be recorded including severity, seriousness and causality;
- Proportion of participants alive, not being admitted to ICU for invasive or non-invasive ventilation and not having required administration of tocilizumab within 14 days (or time to the endpoint);
- Respiratory failure requiring non-invasive or mechanical ventilation;
- Increase of CRP >100% within 3 days;
- Increase of D-Dimer >100% within 3 days;
- Decrease in SpO₂ >3%;
- Chest infiltrates on day 5 and 14 (>15% increase in infiltrates);
- Time to clinical cure (lung imaging improved, normal body temperature for 2 days, clinical condition improved);
- Length of ICU stay;
- Length of invasive or non-invasive ventilation;
- Time to defervescence (afebrile for 48h);
- Change from baseline of CRP, LDH, D-Dimer, IL-6, and Ferritin;
- Time to virological clearance;
- Time to change in National Early Warning Score 2 scoring system (aim: <3);
- Improvement of lung imaging on day 14 (change from baseline);
- Time to recovery of SO₂ (>93%);
- COVID-19 Viral load on blood sample and nasopharyngeal swab ;
- Changes in plasma levels of the following parameters: IL-6, D-Dimer, LDH, CRP, Triglycerides, Ferritin, Coagulation Factors, CH50, C3, and C4.

Treatment was completed in all patients with no allergic reaction evident. No treatment emerging adverse events attributed to C1INH were recorded by the treating physicians. Measurement of activated partial thromboplastin time (aPTT) remained stable or decreased during treatment with C1INH. Thromboembolic complications were not noted in these patients, despite the observed higher incidence of such complications in critically-ill COVID-19 patients. Patient 2 developed severe acute on chronic renal failure on day 2 with a 2.5x increase in serum creatinine from baseline on day 5 (peak). This was attributed to severe diarrhea secondary to COVID-19 infection and lopinavir/r treatment. After aggressive hydration with Ringer's lactate and sodium bicarbonate solution and cessation of lopinavir/r his renal function returned to almost baseline before discharge.

Surprisingly, in view of the disease level present in the patients at the start of treatment and the normal disease progression in COVID-19 patients, four out the five patients improved significantly within one day of treatment. All monitored parameters normalized during the course of observation. Three patients left the hospital as healed within one week after recombinant human C1INH treatment. All patients recovered within three weeks (range 6-20 days).

### Outcome

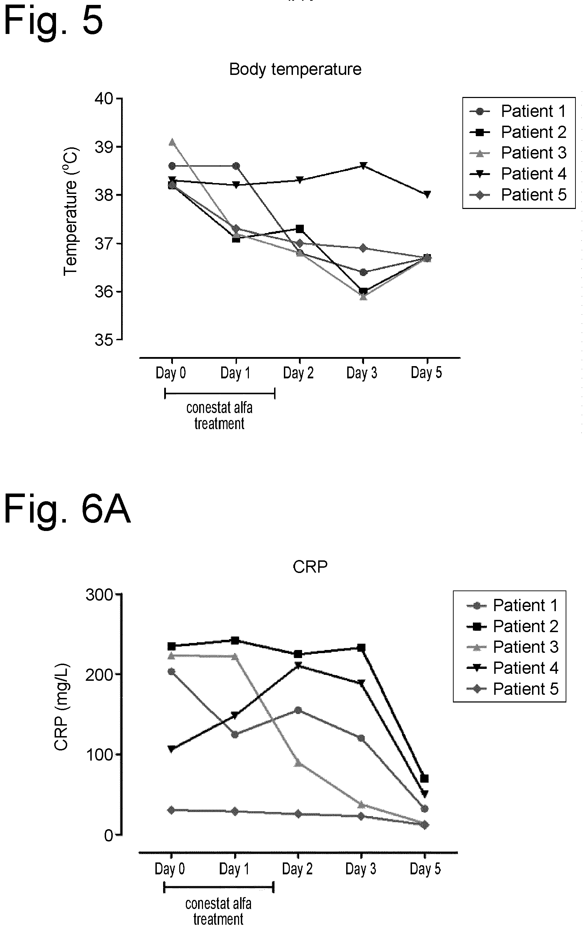

With the exception of patient 4, immediate defervescence was noted in all patients within 48 hours from the start of C1INH treatment. **Figure** 5 shows the temporal change of body temperature following C1INH administration, with day 0 denoting the day of first C1INH administration. In addition, (patients 1 and 5) were weaned off oxygen supplementation after 5 days.

After stabilization of oxygen requirements in patient 2, acute renal failure and subsequent hydration led to increased oxygen requirements secondary to decompensated heart failure on day 4 (increase in NT-proBNP from 1'047 on admission to 39' 156 ng/L), and tocilizumab was administered (despite improvements in LDH, IL-6 and ferritin), which may have confounded any effect of C1INH on respiration. Patient 2 was discharged after renal function was improving.

In patient 3, CIINH was administered late during the admission (day 7) when the patient had massively progressed with a CT scan showing an increase from 7% to 39% pulmonary parenchymal involvement within two days. Hence, only 12 hours after the start of C1INH, tocilizumab was administered twice to save this patient's life. In contrast to several elderly patients with similar lung involvement, who had died within 48 hours despite immediate treatment with tocilizumab, the condition of this patient stabilized over the next days and he was transferred to a rehabilitation facility without oxygen requirements on day 12 after C1INH treatment.

Patient 4 was still febrile on day 2, oxygen requirements were markedly increased (from 2 liters to 8 liters of low-flow nasal cannula oxygenation) and a repeat CT scan showed increased pulmonary parenchymal involvement (from 21 to 36%). Hence, the patient was intubated, and tocilizumab and amoxicillin/clavulanic acid were administered. Subsequently, the patient was extubated after 10 days and finally discharged on day 20.

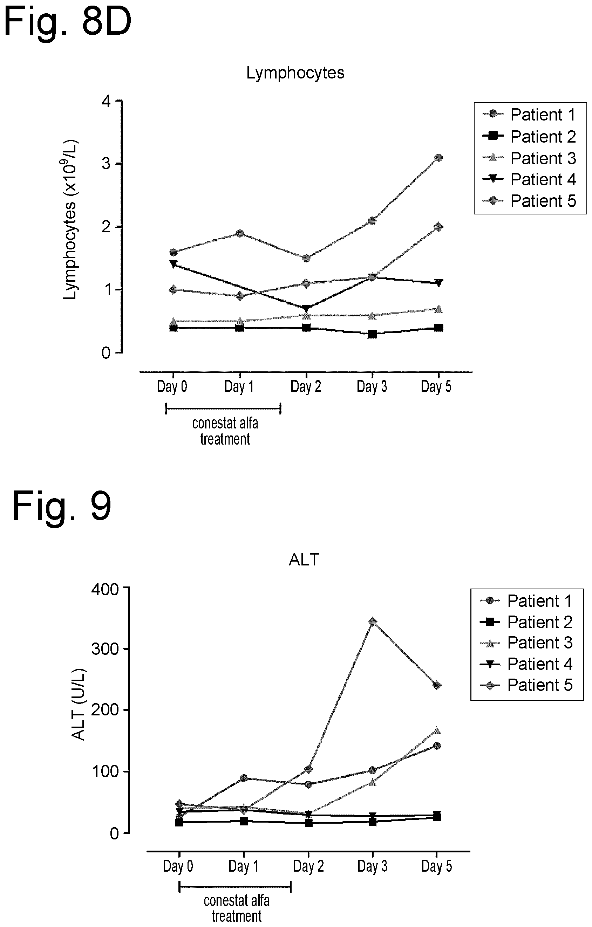

Elevated liver function tests were documented in three patients (see **Figure 9****).** In patient 1, ALT rose to a maximum of 2.5x ULN on day 5, which was attributed to lopinavir/r or amoxicillin/clavulanic acid. In patient 3, ALT rose to a maximum of 2x ULN on day 5, which was attributed to tocilizumab. In patient 5, ALT rose to a maximum of 5x ULN on day 3, which was attributed to lopinarvir/r and paracetamol (concomitantly, LDH and ferritin rose again after an initial decline). A liver ultrasound was unremarkable including the portal vein system. All liver function tests were trending down or had returned to baseline before discharge.

### Laboratory Testing

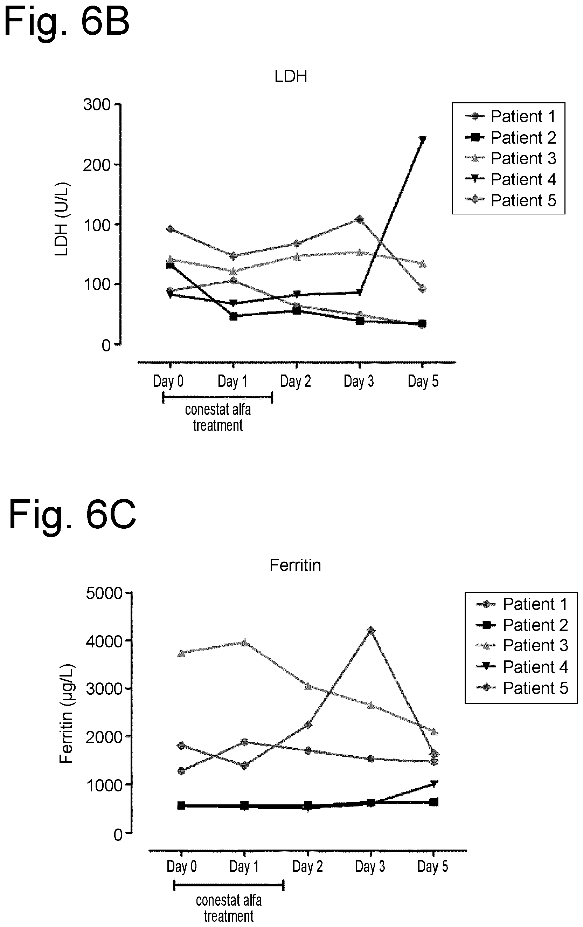

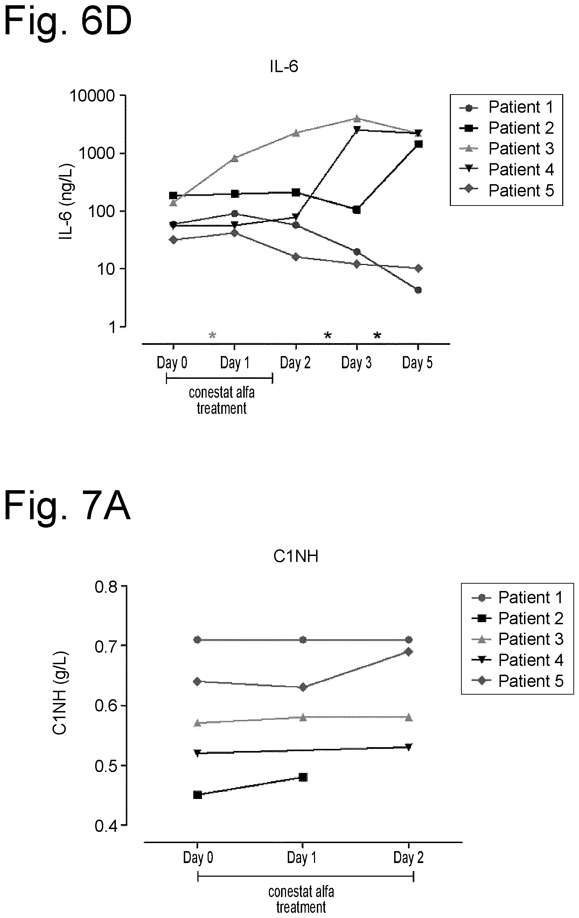

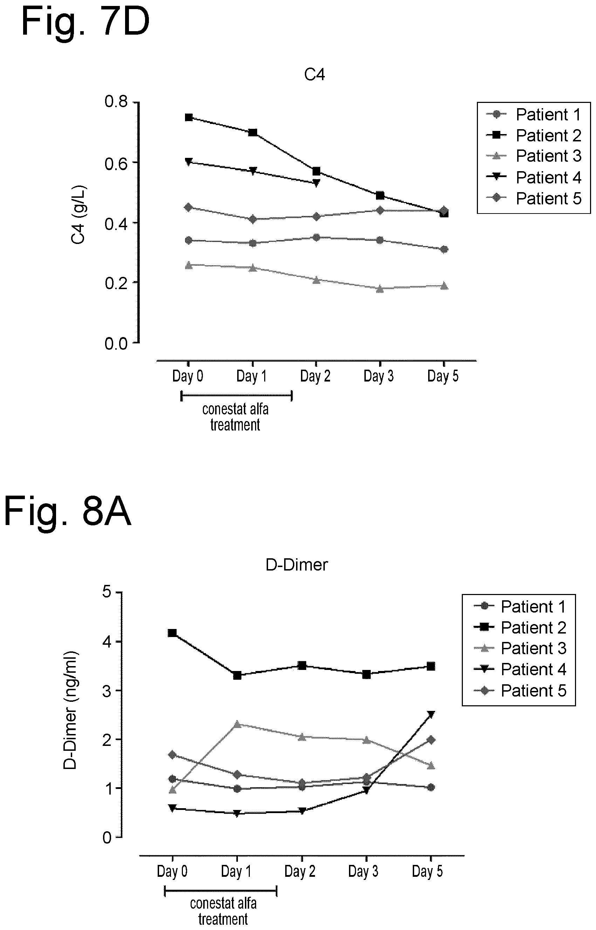

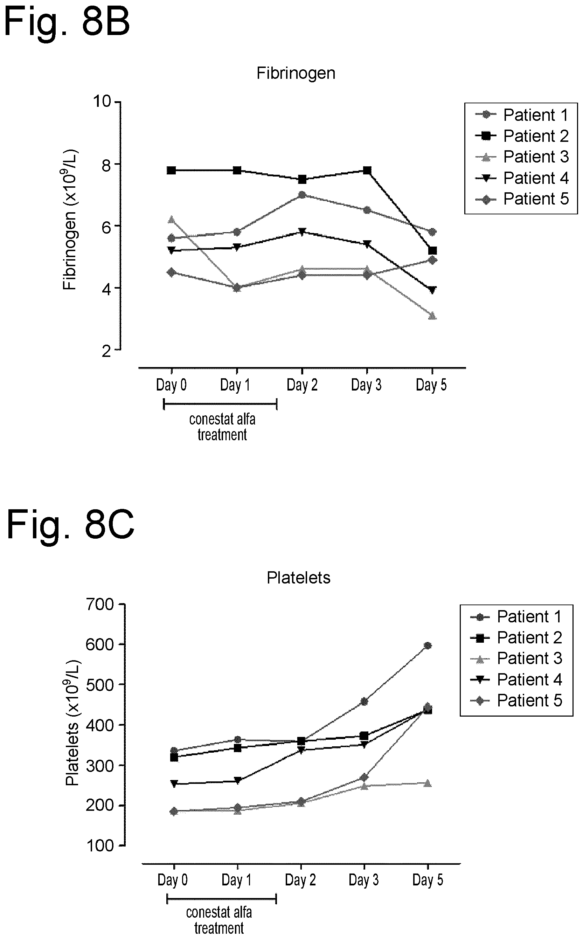

Inflammatory markers were moderately elevated in all patients prior to the treatment with C1INH (see **Figure 1****).** After the treatment, CRP, LDH and ferritin decreased in three patients within 48-72 hours (patients 1, 2, 5) and within 5 days in patient 3 (see **Figures 6A-6C****).** In patient 5, LDH and ferritin increased again on day 2 after an initial decrease, which was most likely attributed to drug-induced liver injury (and not COVID-19 infection). IL-6 also decreased rapidly in patients 1 and 5, whereas it rose dramatically after the administration of tocilizumab in patients 2, 3 and 4 (see **Figure 6D****).** Similarly, blood lymphocytes significantly increased in patients 1 and 5 and remained stable in patients 2 and 3 (see **Figure 8D****).** In contrast, inflammatory markers further increased in patient 4 despite treatment with C1INH (see **Figures 6A-6C****).** Temporal change of D-Dimer **(****Figure 8A****),** fibrinogen **(****Figure 8B****),** and platelets **(****Figure 8C****)** were also monitored.

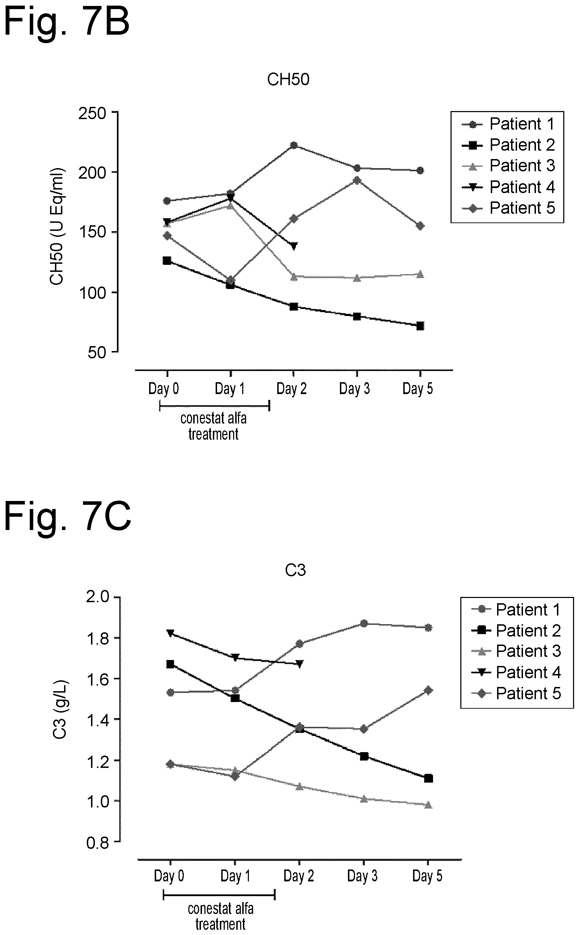

Antigenic C1INH concentration was measured before the first C1INH injection and again prior to the 2^{nd} and 4^{th} injection. C1INH levels were moderately elevated already before the 1^{st} injection in all patients (range, 0.45-0.71 g/L, normal range 0.21-0.39), and remained stable throughout the treatment (see **Figure 7A****).** Interestingly, classical complement pathway activity (CH50) and C3 levels rose in patients 1 and 5, whereas the opposite was observed in the other patients (see **Figures 7B-7C****).** One patient (patient 2) showed a decrease in C4 levels over the course of treatment (see

### Figure 7D).

SARS-CoV-2 viral loads in nasopharyngeal swabs declined in 3/5 patients after 5 days and in 1/5 patient after 10 days.

### Summary

Severe pneumonia caused by SARS-CoV-2 is characterized by fever, elevated proinflammatory markers and lymphopenia. An overreacting immune system is thought to contribute to the clinical picture observed including activation of the complement system and kallikrein system. In this case series, a strategy of complement system and kallikrein system inhibition was pursued for the first time in patients infected with SARS-CoV-2. Five non-critically ill patients with severe COVID-19 pneumonia were treated with C1INH, a potent inhibitor of classical and lectin pathway activation and the kallikrein system, for 48 hours starting a median of one day after hospital admission. Treatment with C1INH over 48 hours was feasible and well tolerated without any treatment emergent adverse events that were attributed to C1INH by the treating physicians. In particular, secondary bacterial infections were not observed. With the exception of one patient, SARS-CoV-2 viral loads declined within 5 and 10 days, respectively, and hence no signal of impaired viral clearance emerged from this case series.

Clinical conditions improved in all but one patient, which was reflected by immediate defervescence, improvement in oxygen requirements and laboratory markers of inflammation in the majority of patients. Importantly, 4/5 patients did not deteriorate and require intensive care unit admission and invasive or non-invasive ventilation. Hence, with the exception of one patient (patient 4) who was mechanically ventilated, outcome was favorable. This is remarkable, given that IL-6 levels were markedly elevated (>80 ng/ml) during the disease course in 4/5 patients, a finding shown to predict respiratory failure and mechanical ventilation in a recent study (Herold T. et al. 2020, see medRxiv preprint at https://doi.org/10.1101/2020.04.01.20047381). Given this data, the use of C1INH to block complement system and kallikrein system activation may be considered in COVID-19 patients.

A relative deficiency of C1INH in some COVID-19 patients may be present facilitating ongoing extensive activation of the complement system and kallikrein system. Consequently, supplementation of C1INH via administration of C1INH is a very plausible treatment option in selected COVID-19 patients. In this regard, it is interesting to note, that complement levels were not elevated in all five patients potentially reflecting consumption in affected organs. Of note, the two patients with the most favorable response (patients 1 and 5) demonstrated a continuous increase in complement C3 and CH50 levels following C1INH administration, which may reflect decreased activation of the CS. In contrast, complement levels decreased in the other patients with a less favorable or delayed response.

Another aspect for future evaluation in clinical trial evolved from the course of disease in patient 3. This patient deteriorated rapidly 5 days after admission demonstrating high-grade fevers and an increase of lung involvement from 7 to 39% in only two days. Still, he survived without invasive ventilation after administration of C1INH and tocilizumab, which is notable given that similar patients that did not survive despite the treatment with tocilizumab. Similarly, in published case series of IL-6 inhibition, 20-30% of patients did not survive (Gritti G. et al. 2020, medRxiv preprint https://doi.org/10.1101/2020.04.01.20048561). Tocilizumab is not thought to interfere significantly with complement activation or the kallikrein-kinin ("KK") system. Hence, a combined approach of inhibition of IL-6 signaling and complement KK system activation should be investigated in selected patients with an overwhelming inflammatory response.

Patient selection is important, in particular regarding the stage of inflammation. The treatment time point may be a key factor associated with the efficacy of C1INH. C1INH may perform better as rescue treatment if administered earlier during the disease course. In this regard, a pulmonary parenchymal involvement of more than 20% as seen in patients 3 and 4 may represent a stage of inflammation that is less amenable to C1INH treatment (or only to combined treatment with other inhibitors of the inflammatory cascade).

### Example 2: Clinical Trial Testing the Effects of C1INH in COVID-19 Patients Suffering From Respiratory Distress

To analyse the effects of C1INH on SARS-CoV-2 infection and the resulting COVID-19 disease, a controlled randomized phase 2 clinical study will be performed testing the efficacy of Ruconest^{®}. The study is titled "Recombinant human C1 esterase inhibitor in the prevention of severe SARS-CoV-2 infection in hospitalized patients with COVID-19: a randomized, parallel-group, open-label, multi-center pilot trial (PROTECT-COVID-19)." The study will enroll up to 120 patients who are suffering from a severe COVID-19 infection, and the sample size can be adjusted based on the results of the interim analyses, if necessary.

Pneumonia is the most frequent serious manifestation of COVID-19, characterized primarily by fever, cough, dyspnea and bilateral infiltrates visible on chest imaging. Acute respiratory distress syndrome (ARDS) is a life-threatening complication associated with an aberrant inflammatory reaction triggered by complement activation. C1 esterase inhibitor (C1-INH) is the direct natural inhibitor of both the complement and the lectin pathway activation and is the most potent known natural inhibitor of this inflammation cascade. We hypothesize that administration of Ruconest^{®} will counteract the above mentioned deleterious complement activation during the course of severe Covid-19.

### Objectives

The primary objective of the study is to determine if adding 72 hours of treatment with C1INH to standard of care treatment in adult participants admitted with non-critically ill COVID-19 will affect disease severity within 7 days after enrolment as assessed by the WHO Ordinal Scale for Clinical Improvement. The WHO 7-point outcome scale at day 7 (or time to improvement of one or two category) is:
- Not hospitalized, no limitations on activities;
- Not hospitalized, limitation on activities;
- Hospitalized, not requiring supplemental oxygen;
- Hospitalized, requiring supplemental oxygen;
- Hospitalized, on non-invasive ventilation or high flow oxygen devices;
- Hospitalized, on invasive mechanical ventilation or ECMO;
- Death.

Secondary objectives will be to determine if C1INH will (1) reduce the time to clinical improvement (time from randomisation to an improvement of two points on the WHO ordinal scale or live discharge from hospital, whichever came first) within 14 days after enrolment; (2) increase the proportion of participants alive and not having required invasive or non-invasive ventilation at 14 days after enrolment; or (3) reduce the proportion of subjects with an acute lung injury (defined by PaO₂/FiO₂ ratio of <300mmHg) within 14 days after enrolment.

Other factors that will be observed include:
- Safety: Participants will be monitored for adverse events and these will be recorded including severity, seriousness and causality;
- Proportion of participants alive, not being admitted to ICU for invasive or non-invasive ventilation and not having required administration of tocilizumab within 14 days (or time to the endpoint);
- Respiratory failure requiring non-invasive or mechanical ventilation;
- Increase of CRP >100% within 3 days;
- Increase of D-Dimer >100% within 3 days;
- Decrease in SpO₂ >3% ;
- Chest infiltrates on day 5 and 14 (>15% increase in infiltrates);
- Time to clinical cure (lung imaging improved, normal body temperature for 2 days, clinical condition improved);
- Length of ICU stay;
- Length of invasive or non-invasive ventilation;
- Time to defervescence (afebrile for 48h);
- Changes in biomarker levels until day 14: CRP, LDH, D-dimer, ferritin, IL-6, lymphocyte count
- Time to virological clearance;
- Time to change in National Early Warning Score 2 scoring system (aim: <3);
- Improvement of lung imaging on day 14 (change from baseline);
- Time to recovery of SO₂ (>93%);
- COVID-19 Viral load on blood sample and nasopharyngeal swab;
- Changes in plasma levels of the following parameters: IL-6, D-Dimer, LDH, CRP, Triglycerides, Ferritin, Coagulation Factors, CH50, C3, C4.

### Study Population

Approximately 120 patients suffering from severe COVID-19 will be enrolled. To be eligible, patients must be age 18-85 years, admitted to the hospital because of confirmed (by a positive SARS-CoV-2 PCR result) COVID-19 infection, display evidence of pulmonary involvement on CT scan of the chest, have had symptom onset within the previous 10 days and at least one additional risk factor for progression to mechanical ventilation: 1) arterial hypertension, 2) ≥50 years, 3) obesity (BMI≥30.0 kg/m2), 4) history of cardiovascular disease, 5) chronic pulmonary disease, 7) chronic renal disease, 6) C-reactive protein of >35mg/L, 7) oxygen saturation at rest in ambient air of ≤94%.

Exclusion criteria include contraindications to the class of drugs under study (C1 esterase inhibitor), treatment with tocilizumab or another I1-6R or I1-6 inhibitor before enrolment, a history or suspicion of allergy to rabbits, pregnancy or breast feeding, active or planned treatment with any other complement inhibitor, liver cirrhosis (any Child-Pugh score), currently admitted to an ICU or expected admission within the next 24 hours, currently receiving invasive or non-invasive ventilation, and participation in another study with investigational drug within the 30 days preceding (with exceptions for other COVID-19 studies)

### Treatment

Patients will be randomized between two treatment groups. Group 1 (Standard Treatment plus Ruconest^{®} group) will receive an initial dose 100U/kg Ruconest, and thereafter will receive 50 U/kg Ruconest^{®} every eight hours over a period 72 hours on top of standard of care according to the investigator's discretion. Group 2 (Standard Treatment group) will receive standard of care according to the investigator's discretion.

Ruconest^{®} is purified from the milk of rabbits expressing the gene coding for human C1INH. Ruconest^{®} is supplied as a sterile, preservative-free, white/off-white lyophilized powder for reconstitution for injection. Each vial contains 2100 units of Ruconest^{®}, 937 mg of sucrose, 83.3 mg of sodium citrate dihydrate and 1.0 mg of citric acid monohydrate. One international unit (U) of C1INH activity is defined as the equivalent of C1INH activity present in 1 mL of pooled normal plasma. After reconstitution with 14 mL of sterile water for injection, each vial contains 150 U of Ruconest per 1 mL in a 20 mM sodium citrate buffer with a pH of 6.8; vials are for single use only.

CIINH (Ruconest^{®}) will be administered as slow (5 min for 4200 U dose and 10 min for 8400 U dose) intravenous injection via a peripheral or central intravenous line. A uniform dose of 8400 U (initial dose) and 4200 U (subsequent doses) was chosen irrespective of body weight. The licensed dosage for CIINH is weight-based (50 U/kg up to 84 kg and 4200 U for a bodyweight of > 84 kg). This is based on the aim to at least achieve a level of 0.7 U/ml C1INH in patients (lower limit of normal of C1INH activity). Simulation studies have revealed that this aim is achievable with the licensed dosing. However, these simulations have also shown that C1INH levels will be lower when using 50 U/kg compared to 4200 U in patients with a bodyweight < 84kg. As for the current trial the aim is not to correct underlying absolute C1INH deficiency (as in cases of hereditary angioedema), but to ensure that a level of at least twice the serum concentration will be achieved in the vast majority of patients, a fixed dose for all patients irrespective of body weight will be used.

In patients with normal C1INH levels, the chosen dose will increase plasma C1-inhibitor activities by at least 100% (4200 U) and 200% (8400 U), respectively. To maximise efficacy C1INH will be administered repeatedly over 72 hours. Maximal volume of the injection is 28ml (4200 U) and 56ml (8400 U) per administration, respectively. C1INH will be administered every eight hours (see **Figure 4B****)**

Repeated administration of C1INH was chosen for several reasons. First, hyperinflammation caused by SARS-CoV-2 is a phenomenon that may last for several days, and hence sustained inhibition of the CS and the KK system is required. Second, the elimination half-life of C1INH was determined at 2.5 hours. Third, a decline of C1 inhibitor activity to pre-administration levels was demonstrated within four to six hours after administration of C1INH at a dose of 50 U/kg. Hence, in order to generate supra-physiological C1INH for the majority of time during a 72h interval, administration of a loading dose followed by repeated injections of C1INH was chosen.

Duration of exposure to C1INH will be approximately 74 hours (up to 10 hours after the last dose administered 64 hours after the first dose). Participants will be followed in hospital for at least 12 hours after the last dose and via structured telephone interviews four weeks later.

### Statistical methods

The primary endpoint WHO 7-point outcome scale at Day 7 will be analyzed by nonparametric logrank test stratified by its baseline values with two-sided α-level of 5 %. The main secondary endpoint is time to improvement of at least 2 points. The secondary endpoint will be tested only after a significant test of the primary endpoint (a priori ordered hypotheses). Therefore, no alpha adjustment is necessary.

Two adaptive interim analyses after 40 and 80 patients are planned according to the Pocock adjusted levels αₚ = 0.0221. The results of the sequential groups are combined by the inverse-normal-method (Lehmacher, Wassmer, 1999). There are no prespecified futility margins, but the Data Safety Monitoring Board can stop the study in the case of insufficient interim results.

### Example 3: Clinical Trial Testing the Effects of CLINH in COVID-19 Patients Suffering From Respiratory Distress

To analyse the effects of C1INH on SARS-CoV-2 infection and the resulting COVID-19 disease, a controlled randomized phase 2 clinical study will be performed testing the efficacy of Ruconest^{®}. The study is titled "Recombinant human C1 esterase inhibitor (rhC1INH) (Ruconest^{®}) in the prevention of severe SARS-CoV-2 infection in hospitalized patients with COVID-19: a randomized, parallel-group, open-label, multi-center pilot trial in the United States (PROTECT-COVID-19-US)." The study will enroll approximately 120 patients who are suffering from a severe COVID-19 infection, and two interim analyses after 40 and 80 patients are planned.

Participants will be randomly assigned in a 2:1 ratio to rhC1INH treatment in addition to standard of care (SOC) or SOC stratified by the site. Screening, informed consent and randomization will happen as early as possible after admission to the hospital, usually within 48 hours after admission. Participants will receive intravenous rhC1INH in addition to standard of care (SOC) or SOC during an admission period starting on the day after informed consent (=day 0). Both groups will continue to receive SOC treatment for COVID-19 infection. Blood samples will be collected before and during the 4-day period of treatment. Follow-up will include the period until discharge and a weekly telemedicine interview or telephone call for 3 months post treatment to assess potential adverse events and outcomes.

Pneumonia is the most frequent serious manifestation of COVID-19, characterized primarily by fever, cough, dyspnea and bilateral infiltrates visible on chest imaging. Acute respiratory distress syndrome (ARDS) is a life-threatening complication associated with an aberrant inflammatory reaction triggered by complement activation. C1 esterase inhibitor (C1-INH) is the direct natural inhibitor of both the complement and the lectin pathway activation and is the most potent known natural inhibitor of this inflammation cascade. We hypothesize that administration of Ruconest^{®} will counteract the above mentioned deleterious complement activation during the course of severe Covid-19.

### Objectives

The primary objective of the study is to determine if adding 96 hours (*i.e.,* four days) of treatment with C1INH to standard of care treatment in adult participants admitted with non-critically ill COVID-19 will affect disease severity within 7 days after enrolment as assessed by the WHO Ordinal Scale for Clinical Improvement. The WHO 7-point outcome scale at day 7 (or time to improvement of one or two category) is:
- Not hospitalized, no limitations on activities;
- Not hospitalized, limitation on activities;
- Hospitalized, not requiring supplemental oxygen;
- Hospitalized, requiring supplemental oxygen;
- Hospitalized, on non-invasive ventilation or high flow oxygen devices;
- Hospitalized, on invasive mechanical ventilation or ECMO;
- Death.

Secondary objectives will be to determine if C1INH will (1) reduce the time to clinical improvement (time from randomisation to an improvement of two points on the WHO ordinal scale or live discharge from hospital, whichever came first) within 14 days after enrolment; (2) increase the proportion of participants alive and not having required invasive or non-invasive ventilation at 14 days after enrolment; or (3) reduce the proportion of subjects with an acute lung injury (defined by Berlin severity criteria) within 14 days after enrolment.

Other factors that will be observed include:
- The study will evaluate the safety of rhC1INH in the setting of COVID-19 infections if added to SOC compared to SOC treatment only by measuring the incidence of adverse events up to 3 months post-treatment.
- Changes on the WHO Ordinal Scale from baseline over 14 days.
- Length of hospital stay until day 28 in survivors.
- Proportion of participants progressing to mechanical ventilation on day 7 and day 14.
- Proportion of participants requiring ICU treatment on day 7 and 14.
- Length of ICU stay until day 28.
- Ventilator-free days until day 28.
- All-cause mortality (time from randomization to death within 4 weeks).
- Changes in biomarker levels until day 14: CRP, LDH, D-dimer, ferritin, IL-6, lymphocyte count.
- Time to virological clearance of SARS-CoV-2 by PCR from upper or lower respiratory tract samples (time from enrollment to first of 2 negative assays at least 12 hours apart).
- Proportion of patients receiving additional anti-inflammatory treatment such as tocilizumab or immunoglobulins within 14 days.
- Time to defervescence (temperature <38.0°C sustained for at least 48 hours).
- Time to clinical improvement (defervescence, normalization of oxygen saturation (>93%) and respiratory rate) until day 28.
- Duration of supplemental oxygen until day 28.
- In a subgroup of patients, the pharmacokinetics and pharmacodynamics of rhCIINH in COVID-19 patients will be characterized by measuring the concentration of rhC1INH and the activity of C1-INH and proteins (such as C3, C4).

### Study Population

Approximately 120 patients suffering from severe COVID-19 will be enrolled.

Participants fulfilling all of the following inclusion criteria are eligible for the study:
- Informed Consent as documented by signature;
- Male or Female age 18-85 years;
- Admitted to the hospital because of confirmed COVID-19 infection (by a positive SARS-CoV-2 PCR result);
- Expected to remain an inpatient over the next four (4) calendar days from time of enrollment;
- Evidence of pulmonary involvement on CT scan or chest X-Ray of the chest (e.g. ground glass opacities);
- Symptom onset within the previous 10 days OR shortness of breath within the previous 5 days. Symptoms include fever or one respiratory symptom (patients presenting later may have already progressed to an inflammatory state that is potentially not amenable to C1INH treatment). Respiratory symptoms include cough, sore throat, hemoptysis, shortness of breath, runny nose, or chest pain; and
- at least one additional risk factor for progression to mechanical ventilation: 1) arterial hypertension, 2) >50 years, 3) obesity (BMI>30.0 kg/m2), 4) history of cardiovascular disease, 5) chronic pulmonary disease, 6) chronic renal disease, 7) C-reactive protein of >35mg/L, 8) oxygen saturation at rest in ambient air of <94%. Cardiovascular disease includes a history of coronary artery disease, cerebrovascular disease, peripheral artery disease, rheumatic heart disease, congenital heart disease and of recent (< 3 months) deep vein thrombosis or pulmonary embolism. Chronic pulmonary disease includes a history of chronic obstructive pulmonary disease, asthma, occupational lung disease, interstitial lung disease or of pulmonary hypertension. Chronic renal disease is defined as a history of an estimated glomerular filtration rate (according to the Chronic Kidney Disease Epidemiology Collaboration equation) < 60ml/min/1.73 m2 for at least three months.

The presence of any one of the following exclusion criteria will lead to exclusion of the participant:
- Contraindications to the class of drugs under study (C1 esterase inhibitor), e.g. known hypersensitivity or allergy to class of drugs or the investigational product;
- Treatment with tocilizumab or another I1-6R or I1-6 inhibitor (within 24 hours) prior to enrollment;
- History or suspicion of allergy to rabbits;
- Women who are of childbearing potential and not using at least one method of contraception (oral contraceptives, barrier methods, approved contraceptive implant or abstinence) before randomization, after discharge and for the entire follow up study period;
- Women who are pregnant or breast feeding or have a positive serum β-human chorionic gonadotropin (hCG) pregnancy test at screening;
- Active or planned treatment with any other complement inhibitor;
- Chronic Liver Disease (any Child-Pugh score B or C);
- Incapacity or inability to provide informed consent;
- Currently admitted to an ICU or expected admission within the next 24 hours;
- Currently receiving invasive or non-invasive ventilation, (with the exception of high-flow oxygen therapy);
- In the opinion of the treating time, death is deemed to be imminent and inevitable within the next 24 hours;
- Participation in another study with investigational drug within the 30 days preceding and during the present study with the following exemptions:
   1) participation in COVID-19 drug trials started at least 48 hours before admission (e.g. post-exposure prophylaxis with hydroxychloroquine) and
   2) participation in COVID-19 drug trials during ICU admission;
- Any uncontrolled or significant concurrent illness that would put the patient at a greater risk or limit compliance with the study requirements at the discretion of the investigator;
- Previous enrollment into the current study; and
- Enrollment of the Investigator, his/her family members, employees and other dependent persons.

Subjects may voluntarily withdraw from study participation at any time without having to provide a reason. Subjects may be withdrawn because of the appearance of a new health condition requiring care or medications prohibited by the protocol, unacceptable adverse event, refusal to continue treatment, or at the Investigator's discretion if it is in the subject's best interest. A subject who withdraws informed consent before randomization or who develops a violation of the selection criteria before randomization is defined as a screening failure. No follow-up of screening failures will be performed. Participants who withdraw informed consent, who do not fulfil inclusion/exclusion criteria after obtaining informed consent or who are diagnosed with an alternative disease (e.g. influenza infection) and have not received any study medication will be withdrawn from the study. Participants who experience a type I allergic reaction after any dose of study medication will be discontinued from further study interventions. Withdrawn or discontinued participants will not be replaced.

### Treatment

Patients will be randomized between two treatment groups. Group 1 (Standard Treatment plus Ruconest^{®} group) will receive Ruconest^{®} on top of standard of care according to the investigator's discretion. Group 2 (Standard Treatment group) will receive standard of care according to the investigator's discretion.

RhC1INH (Ruconest^{®}) will be supplied by the production company Pharming Technologies, B.V., the Netherlands. RhC1INH is a recombinant analogue of human C1-INH for intravenous injection. The primary and secondary structures of the molecule and target protease selectivity are consistent with those of plasma-derived C1-INH. Ruconest^{®} is purified from the milk of rabbits expressing the gene coding for human C1INH. Ruconest^{®} is supplied as a sterile, preservative-free, white/off-white lyophilized powder for reconstitution for injection. Each vial contains 2100 units of Ruconest^{®}, 937 mg of sucrose, 83.3 mg of sodium citrate dihydrate and 1.0 mg of citric acid monohydrate. One international unit (U) of C1INH activity is defined as the equivalent of C1INH activity present in 1 mL of pooled normal plasma. After reconstitution with 14 mL of sterile water for injection, each vial contains 150 U of Ruconest per 1 mL in a 20 mM sodium citrate buffer with a pH of 6.8; vials are for single use only.

After randomization, a pharmacist will open the respective sealed boxes, will reconstitute the respective number of rhCIINH vials and prepare the study medication. RhC1INH is for intravenous use only. The reconstituted solution is administered as a slow intravenous injection over approximately five minutes. Recommended doses of rhC1INH for the treatment of an acute angioedema attack are 50 U/kg if body weight <84kg and 4200 U if >84kg.

Ruconest^{®} will be administered at (150 U/ml) at a 50 U/kg dose (max dose of 4200 U) as a slow intravenous injection via a peripheral or central intravenous line in approximately 5 minutes every 12 hours; for 4 days. A total of 8 doses will be administered. (The licensed dosage for rhC1INH is weight-based (50 U/kg up to 84 kg and 4200 U for a bodyweight of > 84 kg) (See **Figure** 4C). This is based on the aim to at least achieve a level of 0.7 U/ml C1-INH in patients with hereditary angioedema (lower limit of normal of C1-INH activity).

However, these simulations have also shown that C1-INH levels will be lower when using 50 U/kg compared to 4200 U in patients with a bodyweight < 84kg. As for the current trial the aim is not to correct underlying absolute C1-INH deficiency (as in case of hereditary angioedema), but to ensure that a level of at least twice the serum concentration will be achieved in the vast majority of patients, we will use the licensed dose.

In patients with normal C1-INH levels, the chosen dose will increase plasma C1-inhibitor activities by at least 100% (4200 U) respectively. To maximize efficacy rhC1INH will be administered repeatedly over 4 days. Maximal volume of the injection is 28ml (4200 U) per administration.

Repeated administration of rhC1INH was chosen for several reasons. First, hyperinflammation caused by SARS-CoV-2 is a phenomenon that may last for several days, and hence sustained inhibition of the CS and the KK system is required. Second, the elimination half-life of rhC1INH was 2.5 hours. Third, a decline of C-INH activity to pre-administration levels was demonstrated within four to six hours after administration of rhCHNH at a dose of 50 U/kg.

Duration of exposure to rhC1INH will be approximately 4 days. Participants will be followed in hospital for at least 12 hours after the last dose and via structured telephone interviews four weeks later.

### Statistical methods

The primary endpoint WHO 7-point outcome scale at Day 7 will be analyzed by nonparametric logrank test stratified by its baseline values with two-sided α-level of 5 %. The secondary endpoint is time to improvement of at least 2 points. The secondary endpoint will be tested only after a significant test of the primary endpoint (a priori ordered hypotheses). Therefore, no alpha adjustment is necessary.

Two adaptive interim analyses after 40 and 80 patients are planned according to the Pocock adjusted levels αₚ = 0.0221. The results of the sequential groups are combined by the inverse-normal-method (Lehmacher, Wassmer, 1999). There are no prespecified futility margins, but the Data Safety Monitoring Board can stop the study in the case of insufficient interim results.

Having now fully described this invention, it will be understood by those of ordinary skill in the art that the same can be performed within a wide and equivalent range of conditions, formulations, and other parameters without affecting the scope of the invention or any embodiment thereof.

Other aspects of the invention will be apparent to those skilled in the art from consideration of the specification and practice of the invention disclosed herein. It is intended that the specification and examples be considered as exemplary only, with a true scope of the invention being indicated by the following claims

## Claims

1. A therapeutically effective amount of C1 esterase inhibitor (C1INH), for use in the treatment of a patient suffering from respiratory distress, wherein the respiratory distress is related to a coronavirus infection.

2. The therapeutically effective amount of C1 esterase inhibitor (C1INH) for use according to claim 1, wherein the respiratory distress is related to COVID-19.

3. The therapeutically effective amount of C1 esterase inhibitor (C1INH) for use according to claim 1 or 2, wherein the patient requires a ventilator.

4. The therapeutically effective amount of C1 esterase inhibitor (C1INH) for use according to any one of claims 1 to 3, wherein the C1INH is administered before the patient requires a ventilator.

5. The therapeutically effective amount of C1 esterase inhibitor (C1INH) for use according to any one of claims 1 to 4, wherein the C1INH has an amino acid sequence identical or similar to the amino acid sequence of endogenous human CI esterase inhibitor.

6. The therapeutically effective amount of C1 esterase inhibitor (C1INH) for use according to any one of claims 1 to 5, wherein the C1INH as a plasma half-life of less than 6 hours.

7. The therapeutically effective amount of C1 esterase inhibitor (C1INH) for use according to any one of claims 1 to 6, wherein the C1INH is plasma-derived human CI esterase inhibitor.

8. The therapeutically effective amount of C1 esterase inhibitor (C1INH) for use according to any one of claims 1 to 7, wherein the C1INH is administered at a dose of at least 25 U/kg body weight of the patient.

9. The therapeutically effective amount of C1 esterase inhibitor (C1INH) for use according to any one of claims 1 to 8, wherein the C1INH is administered about every twelve hours until the clinical symptoms and the inflammatory markers have decreased below 50% of the initial pathological status or reached normal values.

10. The therapeutically effective amount of C1 esterase inhibitor (C1INH) for use according to claim 9, wherein the clinical symptoms are selected from the group consisting of oxygen requirements, radiographic signs, respiratory rate, and a combination thereof.

11. The therapeutically effective amount of C1 esterase inhibitor (C1INH) for use according to any one of claims 1 to 8, wherein the C1INH is administered about every eight hours until the clinical symptoms and the inflammatory markers have decreased below 50% of the initial pathological status or reached normal values.

12. The therapeutically effective amount of C1 esterase inhibitor (C1INH) for use according to any one of claims 1 to 8, wherein the C1INH is administered about every twelve hours until the clinical symptoms and the inflammatory markers have decreased below 50% of the initial pathological status or reached normal values.

13. The therapeutically effective amount of C1 esterase inhibitor (C1INH) for use according to claim 11 or 12, wherein the clinical symptoms are selected from the group consisting of oxygen requirements, radiographic signs, respiratory rate, and a combination thereof.

14. The therapeutically effective amount of C1 esterase inhibitor (C1INH) for use according to any one of claims 11 to 13, wherein the inflammatory markers are selected from the group consisting of CRP, D-dimers, IL-6, ferritin, and a combination thereof.

15. The therapeutically effective amount of C1 esterase inhibitor (C1INH) for use according to any one of claims 1 to 14, wherein the patient is administered one or more therapeutics in addition to C1INH.

## Patentansprüche

1. Therapeutisch wirksame Menge an C1-Esterase-Inhibitor (C1INH) zur Verwendung bei der Behandlung eines Patienten, der an Atemnot leidet, wobei die Atemnot mit einer Coronavirus-Infektion zusammenhängt.

2. Therapeutisch wirksame Menge an C1-Esterase-Inhibitor (C1INH) zur Verwendung nach Anspruch 1, wobei die Atemnot mit COVID-19 zusammenhängt.

3. Therapeutisch wirksame Menge an C1-Esterase-Inhibitor (C1INH) zur Verwendung nach Anspruch 1 oder 2, wobei der Patient ein Beatmungsgerät benötigt.

4. Therapeutisch wirksame Menge an C1-Esterase-Inhibitor (C1INH) zur Verwendung nach einem der Ansprüche 1 bis 3, wobei der C1INH verabreicht wird, bevor der Patient ein Beatmungsgerät benötigt.

5. Therapeutisch wirksame Menge an C1-Esterase-Inhibitor (C1INH) zur Verwendung nach einem der Ansprüche 1 bis 4, wobei der C1INH eine Aminosäuresequenz aufweist, die mit der Aminosäuresequenz des endogenen menschlichen C1-Esterase-Inhibitors identisch oder ihr ähnlich ist.

6. Therapeutisch wirksame Menge an C1-Esterase-Inhibitor (C1INH) zur Verwendung nach einem der Ansprüche 1 bis 5, wobei der C1INH eine Plasmahalbwertszeit von weniger als 6 Stunden aufweist.

7. Therapeutisch wirksame Menge an C1-Esterase-Inhibitor (C1 INH) zur Verwendung nach einem der Ansprüche 1 bis 6, wobei der C1 INH ein aus Plasma gewonnener menschlicher C1-Esterase-Inhibitor ist.

8. Therapeutisch wirksame Menge an C1-Esterase-Inhibitor (C1INH) zur Verwendung nach einem der Ansprüche 1 bis 7, wobei der C1INH in einer Dosis von mindestens 25 U/kg Körpergewicht des Patienten verabreicht wird.

9. Therapeutisch wirksame Menge an C1-Esterase-Inhibitor (C1INH) zur Verwendung nach einem der Ansprüche 1 bis 8, wobei der C1INH etwa alle zwölf Stunden verabreicht wird, bis die klinischen Symptome und die Entzündungsmarker unter 50 % des anfänglichen pathologischen Status gesunken sind oder normale Werte erreicht haben.

10. Therapeutisch wirksame Menge an C1-Esterase-Inhibitor (C1INH) zur Verwendung nach Anspruch 9, wobei die klinischen Symptome aus der Gruppe ausgewählt sind, die aus Sauerstoffbedarf, radiologischen Zeichen, Atemfrequenz und einer Kombination davon besteht.

11. Therapeutisch wirksame Menge an C1-Esterase-Inhibitor (C1INH) zur Verwendung nach einem der Ansprüche 1 bis 8, wobei der C1INH etwa alle acht Stunden verabreicht wird, bis die klinischen Symptome und die Entzündungsmarker unter 50 % des anfänglichen pathologischen Status gesunken sind oder normale Werte erreicht haben.

12. Therapeutisch wirksame Menge an C1-Esterase-Inhibitor (C1INH) zur Verwendung nach einem der Ansprüche 1 bis 8, wobei der C1INH etwa alle zwölf Stunden verabreicht wird, bis die klinischen Symptome und die Entzündungsmarker unter 50 % des anfänglichen pathologischen Status gesunken sind oder normale Werte erreicht haben.

13. Therapeutisch wirksame Menge an C1-Esterase-Inhibitor (C1INH) zur Verwendung nach Anspruch 11 oder 12, wobei die klinischen Symptome aus der Gruppe ausgewählt sind, die aus Sauerstoffbedarf, radiologischen Zeichen, Atemfrequenz und einer Kombination davon besteht.

14. Therapeutisch wirksame Menge an C1-Esterase-Inhibitor (C1INH) zur Verwendung nach einem der Ansprüche 11 bis 13, wobei die Entzündungsmarker aus der Gruppe ausgewählt sind, die aus CRP, D-Dimeren, IL-6, Ferritin und einer Kombination davon besteht.

15. Therapeutisch wirksame Menge an C1-Esterase-Inhibitor (C1INH) zur Verwendung nach einem der Ansprüche 1 bis 14, wobei dem Patienten zusätzlich zu dem C1INH ein oder mehrere Therapeutika verabreicht werden.

## Revendications

1. Quantité thérapeutiquement efficace d'inhibiteur de C1 estérase (C1INH), pour l'utilisation dans le traitement d'un patient souffrant de détresse respiratoire, où la détresse respiratoire est liée à une infection par un coronavirus.

2. Quantité thérapeutiquement efficace d'inhibiteur de C1 estérase (C1INH) pour l'utilisation selon la revendication 1, où la détresse respiratoire est liée au COVID-19.

3. Quantité thérapeutiquement efficace d'inhibiteur de C1 estérase (C1INH) pour l'utilisation selon la revendication 1 ou 2, où le patient nécessite un ventilateur.

4. Quantité thérapeutiquement efficace d'inhibiteur de C1 estérase (C1INH) pour l'utilisation selon l'une quelconque des revendications 1 à 3, où le C1INH est administré avant que le patient ne nécessite un respirateur.

5. Quantité thérapeutiquement efficace d'inhibiteur de C1 estérase (C1INH) pour l'utilisation selon l'une quelconque des revendications 1 à 4, où le C1INH a une séquence d'acides aminés identique ou similaire à la séquence d'acides aminés de l'inhibiteur de la C1 estérase humaine endogène.

6. Quantité thérapeutiquement efficace d'inhibiteur de C1 estérase (C1INH) pour l'utilisation selon l'une quelconque des revendications 1 à 5, où la C1INH a une demi-vie plasmatique inférieure à 6 heures.

7. Quantité thérapeutiquement efficace d'inhibiteur de C1 estérase (C1INH) pour l'utilisation selon l'une quelconque des revendications 1 à 6, où le C1INH est un inhibiteur de la C1 estérase humaine dérivé du plasma.

8. Quantité thérapeutiquement efficace d'inhibiteur de C1 estérase (C1INH) pour l'utilisation selon l'une quelconque des revendications 1 à 7, où le C1INH est administré à une dose d'au moins 25 U/kg de poids corporel du patient.

9. Quantité thérapeutiquement efficace d'inhibiteur de C1 estérase (C1INH) pour l'utilisation selon l'une quelconque des revendications 1 à 8, où le C1INH est administré environ toutes les douze heures jusqu'à ce que les symptômes cliniques et les marqueurs inflammatoires aient diminué en dessous de 50 % de l'état pathologique initial ou atteint des valeurs normales.

10. Quantité thérapeutiquement efficace d'inhibiteur de C1 estérase (C1INH) pour l'utilisation selon la revendication 9, où les symptômes cliniques sont sélectionnés dans le groupe consistant en des besoins en oxygène, des signes radiographiques, la fréquence respiratoire et en une combinaison de ceux-ci.

11. Quantité thérapeutiquement efficace d'inhibiteur de C1 estérase (C1INH) pour l'utilisation selon l'une quelconque des revendications 1 à 8, où le C1INH est administré environ toutes les huit heures jusqu'à ce que les symptômes cliniques et les marqueurs inflammatoires aient diminué en dessous de 50 % de l'état pathologique initial ou atteint des valeurs normales.

12. Quantité thérapeutiquement efficace d'inhibiteur de C1 estérase (C1INH) pour l'utilisation selon l'une quelconque des revendications 1 à 8, où le C1INH est administré environ toutes les douze heures jusqu'à ce que les symptômes cliniques et les marqueurs inflammatoires aient diminué en dessous de 50 % de l'état pathologique initial ou atteint des valeurs normales.

13. Quantité thérapeutiquement efficace d'inhibiteur de C1 estérase (C1INH) pour l'utilisation selon la revendication 11 ou 12, où les symptômes cliniques sont sélectionnés dans le groupe consistant en des besoins en oxygène, des signes radiographiques, la fréquence respiratoire et en une combinaison de ceux-ci.

14. Quantité thérapeutiquement efficace d'inhibiteur de C1 estérase (C1INH) pour l'utilisation selon l'une quelconque des revendications 11 à 13, où les marqueurs inflammatoires sont sélectionnés dans le groupe consistant en CRP, des D-dimères, de l'IL-6, de la ferritine et en une combinaison de ceux-ci.

15. Quantité thérapeutiquement efficace d'inhibiteur de C1 estérase (C1INH) pour l'utilisation selon l'une quelconque des revendications 1 à 14, où le patient est administré une ou plusieurs thérapies en plus du C1INH.
